# EUROPEAN PATENT APPLICATION

(11) **EP 3 981 472 A1**
(43) Date of publication of application: **13.04.2022**
(21) Application number: 20819085.0
(22) Date of filing: 22.05.2020
(51) Int. Cl.: A61P 25/28, A23L 33/10, A23L 33/105, A61K 31/7048

(54) **NEUROLOGICAL FUNCTION REGULATING AGENT**

(30) Priority: 05.06.2019 JP 2019105644
(71) Applicant: University of Tsukuba, Ibaraki 305-8577 (JP); NATIONAL INSTITUTE OF ADVANCED INDUSTRIAL SCIENCE AND TECHNOLOGY, Chiyoda-ku Tokyo 100-8921 (JP); Zeon Corporation, Tokyo 100-8246 (JP)
(72) Inventor: ISODA Hiroko, Tsukuba-shi, Ibaraki 305-8577 (JP); SASAKI Kazunori, Tsukuba-shi, Ibaraki 305-8577 (JP); SATO Kazuhiko, Tsukuba-shi, Ibaraki 305-8561 (JP); ASAKAWA Masumi, Tsukuba-shi, Ibaraki 305-8561 (JP); TOMINAGA Kenichi, Tsukuba-shi, Ibaraki 305-8561 (JP); FUJISAWA Hiroshi, Tokyo 100-8246 (JP); KOSHIYAMA Masami, Tokyo 100-8246 (JP)
(74) Representative: Gerauer, Marc Philippé
(86) International application number: PCT/JP2020/020359
(87) International publication number: WO 2020/246271

(57) **Abstract**

Provided is a neuronal function modulating agent effective for neuronal cell protection and exerts a neuronal function modulating effect through neuronal cell protection. The disclosed neuronal function modulating agent comprises malvidin-3,5-diglucoside as an active ingredient, the disclosed pharmaceutical composition comprises the neuronal function modulating agent, and the disclosed food composition comprises the neuronal function modulating agent.

## Description

### TECHNICAL FIELD

The present disclosure relates to neuronal function modulating agents that comprise a specific malvidin glycoside as an active ingredient.

### BACKGROUND

Recently, active research has been carried out on anthocyanins, active ingredients of natural origin. Anthocyanins have a variety of physiological activities including visual disturbance improving effects, antioxidant effects, vascularization reinforcing effects, anti-inflammatory effects and antitumor effects, depending on the structure of the aglycone as well as on the types and numbers of glycans. Among anthocyanins, malvidin-3,5-diglucoside is claimed to have effects on suppressing cell death caused by ultraviolet rays or active oxygen generated by ultraviolet rays. Topical skin agents have been reported as a specific example of the application of malvidin-3,5-diglucoside (PTL 1).

### CITATION LIST

### Patent Literature

PTL1: JP2004-359603A

### SUMMARY

### (Technical Problem)

Malvidin-3,5-diglucoside is considered safe and suitable for daily or continuous ingestion because it is available as an ingredient of plant origin (biological resource-derived ingredient). It is desirable to find new properties of malvidin-3,5-diglucoside and apply it to medicine and other related fields based on the new properties.

### (Solution to Problem)

As a result of extensive studies, the inventors have established that malvidin-3,5-diglucoside is effective for neuronal cell protection and exerts neuronal function modulating effects through neuronal cell protection. The inventors thus have completed the present disclosure.

According to the present disclosure, there is provided a neuronal function modulating agent that comprises malvidin-3,5-diglucoside as an active ingredient.

The term "neuronal function modulating agent" as used herein refers an agent having a neuronal function modulating effect, and the term "neuronal function modulating effect" as used herein refer to an effect related to the prevention of deterioration , retention, restoration, improvement, or prevention of diseases related to a neural function.

Malvidin-3,5-diglucoside is represented by the following formula (1):

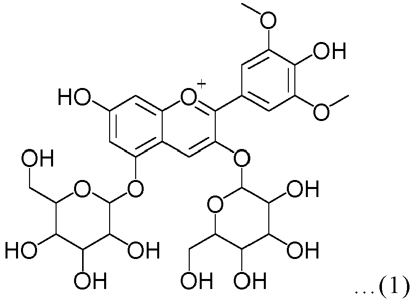

Malvidin-3,5-diglucoside may be in the form having a counter anion, e.g., chloride.

According to the present disclosure, there is provided the neuronal function modulating agent described above that exerts neuronal function modulating effects through neuronal cell protection, and further, there is provided the neuronal function modulating agent described above wherein the neuronal cell protection comprises suppression of neuronal cell death caused by amyloid β protein.

According to the present disclosure, there is provided the neuronal function modulating agent described above that comprises delphinidin-3-glucoside.

According to the present disclosure, there is provided the neuronal function modulating agent described above, which is for oral administration.

According to the present disclosure, there is also provided a pharmaceutical composition that comprises the neuronal function modulating agent described above.

According to the present disclosure, there is also provided a food composition that comprises the neuronal function modulating agent described above, and further, there is provided the food composition described above which is a functional food, a dietary supplement, a health supplement, a nutritionally fortified food, a food with a functional claim, a food for special dietary use, a food for specified health use, or a food with a nutritional function.

According to the present disclosure, there is also provided a method of modulating a neuronal function, which comprises ingesting the neuronal function modulating agent described above into a subject.

### (Advantageous Effect)

According to the present disclosure, there is provided a neuronal function modulating agent effective for neuronal cell protection and exerts neuronal function modulating effects through neuronal cell protection, and also provides a pharmaceutical composition and a food composition which comprise the neuronal function modulating agent.

### BRIEF DESCRIPTION OF THE DRAWINGS

In the accompanying drawings:
FIG. 1 illustrates a graph showing the results of a cell survival experiment in Example 1;
FIG. 2 illustrates graphs showing the results of the expression of MAP2K4 (mitogen-activated protein kinase 4) in the gene expression experiment in Example 2, where the graph (a) shows results with and without malvidin-3,5-diglucoside, and the graph (b) shows the results with malvidin, malvidin-3-glucoside, and malvidin-3,5-diglucoside (the same applies to FIGS. 3 to 8);
FIG. 3 illustrates graphs showing the results of the expression of MAPK14 (mitogen-activated protein kinase 14) in the gene expression experiment in Example 2;
FIG. 4 illustrates graphs showing the results of the expression of MAPK8 (mitogen-activated protein kinase 8) in the gene expression experiment in Example 2;
FIG. 5 illustrates graphs showing the results of the expression of PI3KCA (phosphatidylinositol-4,5-bisphosphate 3-kinase catalyst subunit α) in the gene expression experiment in Example 2;
FIG. 6 illustrates graphs showing the results of the expression of AKT1 (AKT serine/threonine kinase 1) in the gene expression experiment in Example 2;
FIG. 7 illustrates graphs showing the results of the expression of PARP1 (poly(ADP-ribose) polymerase 1) in the gene expression experiment in Example 2;
FIG. 8 illustrates graphs showing the results of the expression of CASP3 (caspase-3) in the gene expression experiment in Example 2;
FIG. 9 illustrates a graph showing the results of the ATP production measurement experiment in Example 3;
FIG. 10 illustrates a graph showing the results of the reactive oxygen species measurement experiment in Example 4;
FIG. 11 illustrates a graph showing the results of a platform test (platform arrival time) in the animal experiment in Example 5.
FIG. 12 illustrates a graph showing the results of a probe test (the number of times to cross the former platform location) in the animal experiment in Example 5; and
FIG. 13 illustrates a graph showing the results of a probe test (time spent on the former platform location) in the animal experiment in Example 5.

### DETAILED DESCRIPTION

The present disclosure will be described in detail below.

### <Neuronal Function Modulating Agent>

The present disclosure relates to a neuronal function modulating agent that comprises malvidin-3,5-diglucoside as an active ingredient.

### (Source)

Malvidin-3,5-diglucoside, also referred to as Malvin, is a type of naturally-occurring anthocyanins and is known as the major pigment of the genus *Malva sylvestris,* the genus *Primula,* and the genus *Rhodoendron.* The neuronal function modulating agent can comprise malvidin-3,5-diglucoside derived from any of these plants. However, malvidin-3,5-diglucoside derived from other biological resources containing this compound can also be used. Further, malvidin-3,5-diglucoside may be isolated from a biological resource or may be used as such without being isolated from the biological resource. For example, malvidin-3,5-diglucoside may be provided as an extract from a biological resource.

Biological resources of malvidin-3,5-diglucoside can be grapes. Grapes belonging to the genus *Vitis* are preferred. Preferred from the viewpoint of the malvidin-3,5-diglucoside content are *Vitis Vinifera, Vitis Coignetiae, Vitis flexuosa,* and their cross species, with *Vitis Coignetiae, Vitis flexuosa,* a cross species between *Vitis Coignetiae* and *Vitis Vinifera,* or a cross species between *Vitis flexuosa* and *Vitis Vinifera* being more preferred.

Because malvidin-3,5-diglucoside can be included in the pericarp of a grape, a ground grape pericarp or a grape pericarp extract can be a source of malvidin-3,5-diglucoside. Any extraction method can be used and extraction can be accomplished for example by the use of an organic solvent. It is preferred to use a grape pericarp extract because anthocyanins other than malvidin-3,5-diglucoside (e.g., delphinidin-3-glucoside, malvidin-3-glucoside, malvidin-3-coumaroylglucoside, and malvidin-3-coumaroylglucoside-5-glucoside) that may be included in a grape pericarp can be obtained efficiently and simultaneously with malvidin-3,5-diglucoside to afford a neuronal function modulating agent that comprises these anthocyanins together with malvidin-3,5-diglucoside.

Commercially available grape powders or grape concentrates may be used as a source of malvidin-3,5-diglucoside.

Malvidin-3,5-diglucoside can be a synthetic product. However, malvidin-3,5-diglucoside is preferably derived from a biological resource from the viewpoint of safety.

### (Effect)

It has been established by the inventors that malvidin-3,5-diglucoside is effective for neuronal cell protection. Neuronal cell protection includes suppression of neuronal cell death. Neuronal cell proliferation may also be observed along with the suppression of neuronal cell death. While the mechanism by which neuronal cells are protected by malvidin-3,5-diglucoside remains elusive, the results of Examples given below suggest both antioxidant effects and energy metabolism-promoting effects are involved.

The neuronal function modulating agent of the present disclosure comprises malvidin-3,5-diglucoside as an active ingredient and may comprise other ingredient(s). Examples of other ingredients include anthocyanins other than malvidin-3,5-diglucoside, e.g., delphinidin-3-glucoside, malvidin-3-glucoside, malvidin-3-coumaroylglucoside, malvidin-3-coumaroylglucoside-5-glucoside.

### (Diseases and conditions to be treated, etc.)

Because the neuronal function modulating agent of the present disclosure exerts a neuronal function modulating function through neuronal cell protection, it is effective for neurodegenerative diseases and other conditions related to neuronal cell damage and/or reduction. Among other diseases and conditions, the neuronal function modulating agent is highly effective for anti-aging. In Examples described below, prevention of deterioration, and improvement, of spatial learning and memory were observed for senescence accelerated model mice with accumulated amyloid β protein. The neuronal function modulating agent is thus effective for neurodegenerative diseases and various conditions that are considered to be caused for example by amyloid β protein or oxidative stress due to amyloid β protein.

Specifically, the neuronal function modulating agent of the present disclosure can be used for the purpose of improving or preventing neurodegenerative diseases such as Alzheimer's disease, Parkinson's disease and depression, and other conditions.

The neuronal function modulating agent of the present disclosure can also be used for the purpose of anti-stress, anti-fatigue.

Further, the neuronal function modulating agent of the present disclosure can be used for the purpose of restoring or improving neuronal functions such as learning ability, memory ability, and reflection and response ability.

The neuronal function modulating agent of the present disclosure can be an agent for anti-aging, anti-stress, anti-fatigue, learning ability restoration/improvement, memory ability restoration/improvement, reflection and response ability restoration/improvement.

The neuronal function modulating agent of the present disclosure can also be administered to a healthy individual.

### (Administration Regimen)

Examples of subjects to be administered with the neuronal function modulating agent include mammals, e.g., humans, domestic animals (e.g., horses, cows, pigs), pet animals (e.g., dogs, cats), and experimental (test) animals (e.g., mice, rats), with humans being preferred. The dose of the neuronal function modulating agent of the present disclosure can be appropriately determined in view of species, age, body weight, conditions to be treated etc., of the subject. For example, the dose of the neuronal function modulating agent, expressed in the dose of malvidin-3,5-diglucoside, can be 0.008 mg/day or more and 4 mg/day or less per kg body weight of the subject (human). The dose is preferably 0.04 mg/day or more, more preferably 0.08 mg/day or more, and preferably 1.6 mg/day or less, more preferably 0.8 mg/day or less. When the subject is not a human, the dose can be determined appropriately by increasing the decreasing the values described above. Administration may be once or two or more times per day. The administration period can be appropriately determined in view of species, age, body weight, conditions to be treated etc., of the subject, but continuous use is preferred.

### (Method of Modulating Neuronal Function)

The present disclosure relates to a method of modulating a neuronal function, which comprises ingesting the neuronal function modulating agent into a subject. The method can exclude humans from the subject and also can exclude medical practice.

### <Pharmaceutical Composition>

The present disclosure also relates to a pharmaceutical composition that comprises the neuronal function modulating agent. The pharmaceutical composition can be a pharmaceutical or quasi-pharmaceutical agent.

The pharmaceutical composition can comprise a pharmaceutically acceptable ingredient. Examples thereof include carriers, excipients (e.g., lactose, sucrose, dextrin, hydroxypropylcellulose, polyvinylpyrrolidone), disintegrating agents, buffers, emulsifiers, suspending agents, stabilizers, preservatives, solvents (e.g., water, saline, and organic solvents such as ethanol). The pharmaceutical composition can comprise other pharmacological components having a pharmacological activity.

The pharmaceutical composition may be for oral or parenteral administration, but is preferably for oral administration. Dosage forms for oral administration can include powders, tablets, coating tablets, sugar-coated tablets, soft or hard capsules, solutions, emulsions, and suspensions. Dosage forms for parenteral administration can include suppositories, injections, infusions, ointments, creams, and gels.

Diseases and conditions to be treated, administration regimen (subject, dose, period) etc. of the pharmaceutical composition can be the same as those for the neuronal function modulating agent described above. The pharmaceutical composition of the present disclosure can be used as a pharmaceutical composition for neuronal function modulation. Examples include pharmaceutical compositions for anti-aging, anti-stress, anti-fatigue, restoration/improvement of learning ability, restoration/improvement of memory ability, or restoration/improvement of reflection and response ability.

### <Food Composition>

According to the present disclosure, there is provided a food composition that comprises the neuronal function modulating agent described above. The food composition may be a functional food, a dietary supplement, a health supplement, a nutritionally fortified food, a food with a functional claim, a food for special dietary use, a food for specified health use, or a food with a nutritional function.

The food composition can comprise ingredients acceptable in the production of foods. Examples thereof include carriers, excipients (e.g., lactose, sucrose, dextrin, hydroxypropylcellulose, polyvinylpyrrolidone), disintegrating agents, buffers, emulsifiers, suspending agents, stabilizers, preservatives, solvents (e.g., water, saline, and organic solvents such as ethanol). Foods can also comprise proteins, carbohydrates, lipids, vitamins, minerals, organic acids, organic bases, flavors, and other ingredients.

The food composition can be in any form including solids, liquids, mixtures, suspensions, pastes, gels, powders, and capsules. The food composition can be a beverage, confectionery, seasoning, daily dish or processed food. The food composition may also be a supplement.

Diseases and conditions to be treated, administration regimen (subject, period) etc. of the food composition can be the same as those for the neuronal function modulating agent described above. The food composition of the present disclosure can be used as a food composition for neuronal function modulation. Examples include food compositions for anti-aging, anti-stress, anti-fatigue, restoration/improvement of learning ability, restoration/improvement of memory ability, or restoration/improvement of reflection and response ability.

### EXAMPLES

The present disclosure will be described in more detail below with reference to Examples, which however shall not be construed as limiting the scope of the present disclosure.

### <Sample Preparation>

In each experiment, the following was used as malvidin-3,5-diglucoside:
Compound (M3,5G): Malvidin-3,5-diglucoside chloride (FUJIFILM Wako Pure Chemical Industries, Ltd.)
Compound (M): Malvidin chloride (FUJIFILM Wako Pure Chemical Industries, Ltd.)
Compound (M3G): Malvidin-3-glucoside chloride (FUJIFILM Wako Pure Chemical Industries, Ltd.)

### Extract: grape pericarp extract containing malvidin-3,5-diglucoside

### The extract was prepared as described below.

Grapes (varietal name: Fujinoyume, a cross species between Gyojano-mizu *(Vitis flexuosa)* and Merlot *(Vitis Vinifera))* were squeezed and the fruit juice was removed. Pericarps were placed on a stainless steel net cage so as not to come into contact with each other, and dried using a dryer by applying 70°C warm air to give a dried grape pericarp product. To 6 g of the dried grape pericarp product was added 40 mL of 2% formic acid in methanol and water (methanol : ultrapure water : formic acid = 70:28:2 (v/v/v)) and the mixture was stirred (360 rpm) for 12h at room temperature under light-shielded conditions. The obtained extract solution was filtered through filter paper (ADVANTEC quantitative filter paper No.5A, 110 mm), concentrated and lyophilized to give 3 g of grape pericarp extract powder.

The obtained grape peel extract powder was dissolved in methanol and subjected to high-performance liquid chromatography-mass spectrometry (HPLC/MS) to confirm that 86.8 mg of malvidin-3,5-diglucoside, 49.2 mg of delphinidin-3-glucoside, 2.6 mg of malvidin-3 glucoside, 12.6 mg of malvidin-3-coumaroylglucoside, and 51.5 mg of malvidin-3-coumaroylglucoside-5-glucoside were contained per gram of the extract powder. The ingredients were identified and quantified using LC-MS libraries and by comparison with the measured values of standards.

Samples 1 to 6 below were prepared using the compounds and extract prepared above. Samples 1 to 4 were used in the cell survival experiment, gene expression experiment, ATP production measurement experiment, and reactive oxygen species measurement experiment described later, and Samples 5 and 6 were used in the animal experiment described later.
Sample 1: Compound (M3,5G) was dissolved in 70% ethanol solution (ethanol : water = 70:30 (v/v)) and the concentration of compound (M3,5G) in the obtained solution was adjusted to 12.56 µM for use for the respective experiments.
Sample 2: Compound (M) was dissolved in 70% ethanol solution (ethanol : water = 70:30 (v/v)) and the concentration of compound (M) in the obtained solution was adjusted to 12.56 µM for use for the respective experiments.
Sample 3: Compound (M3G) was dissolved in 70% ethanol solution (ethanol : water = 70:30 (v/v)) and the concentration of compound (M3G) in the obtained solution was adjusted to 12.56 µM for use for the respective experiments.
Sample 4: The extract was dissolved in 70% ethanol solution (ethanol : water = 70:30 (v/v)) and the concentration of the extract powder in the obtained solution was adjusted to 100 µg/mL for use for the respective experiments. The concentration of malvidin-3,5-diglucoside was 12.56 µM (calculated as chloride).
Sample 5 : Compound (M3,5G) was dissolved in ultrapure water (Milli-Q^{®} water) and the concentration of compound (M3,5G) in the obtained solution was adjusted to 651 µg/mL for use for the animal experiment.
Sample 6: The extract was dissolved in ultrapure water (Milli-Q^{®} water) and the concentration of the extract powder in the obtained solution was adjusted to 7.5 mg/mL for use for the animal experiment. The concentration of malvidin-3,5-diglucoside in the solution was 651 µg/mL (calculated as chloride).

### <Example 1: Cell Survival Experiment>

Inhibitory effects of malvidin-3,5-diglucoside on neuronal cell death were confirmed by the experiment described below.

Each well of a 96-well plate (BD Biocoat) was seeded with SH-SY5Y cells (model of neuronal cells, ATCC) at a concentration of 2×10⁵ cells/mL (100 µL/well) and the cells were cultured at 37°C and 5%CO₂ for 24h. Culture medium used was F12/DMEM (Gibco) supplemented with 1% non-essential amino acids (Gibco), 15%FBS (Bio West), and 1% penicillin/streptomycin (Lonza).

After 24h, the culture medium was completely removed with an aspirator, 100 µL of culture medium Opti-MEM (Gibco) was added to each well, and the cells were cultured at 37°C and 5%CO₂ for 24h.

Opti-MEM was completely removed with an aspirator, and 110 µ L of MTT reagent (3-(4,5-dimethylthiazol-2-yl)-2,5-diphenyltetrazolium bromide, 5mg/mL) mixed with Opti-MEM (MTT reagent : Opti-MEM = 1:10) was added to each well and the cells were cultured at 37°C and 5%CO₂ for 24h. 100 µL of 10%SDS (sodium dodecyl sulfate) aqueous solution was added to each well and the cells were cultured at 37°C and 5%CO₂ for 24h.

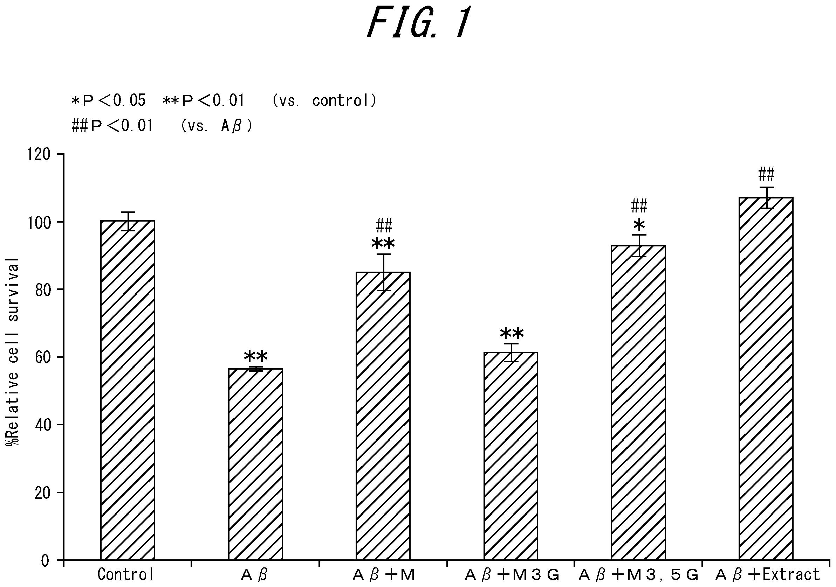

Absorbance at 570 nm was measured on a microplate reader (Sumitomo Dainippon Pharma) to measure the MTT formazan production to calculate %relative cell survival (designated as "Control" in FIG. 1).

24h after seeding a 96-well plate (BD Biocoat) with SH-SY5Y cells, the culture medium was completely removed with an aspirator, amyloid β protein (Beta-Amyloid 1-42, AnaSpec) was mixed with Opti-MEM (Gibco) so that the final concentration was 15 µM, 100 µL of the mixture was added to each well, and the cells were cultured at 37°C and 5%CO₂ for 72h to induce cell death. Except for performing the procedure described above, the MTT formazan production was measured as described above to calculate %relative cell survival (designated as "Aβ" in FIG. 1).

24h after seeding a 96-well plate (BD Biocoat) with SH-SY5Y cells, the culture medium was completely removed with an aspirator, Sample 1 was mixed with Opti-MEM (Gibco) so that the final concentration was 12.56 µM, 90 µL of the mixture was added to each well and allowed to stand for 10 min, amyloid β protein (Beta-Amyloid 1-42, AnaSpec) was mixed with Opti-MEM (Gibco) so that the final concentration was 150 µM, 10 µL of the mixture was added to each well, and the cells were cultured at 37°C and 5%CO₂ for 72h to induce cell death. Except for performing the procedure described above, the MTT formazan production was measured as described above to calculate %relative cell survival (designated as "Aβ + M3,5G" in FIG. 1).

Except that Sample 2 was used instead of Sample 1, %relative cell survival (designated as "Aβ + M" in FIG. 1) was calculated in the same manner as described above.

Except that Sample 3 was used instead of Sample 1, %relative cell survival (designated as "Aβ + M3G" in FIG. 1) was calculated in the same manner as described above.

24h after seeding a 96-well plate (BD Biocoat) with SH-SY5Y cells, the culture medium was completely removed with an aspirator, Sample 4 was mixed with Opti-MEM (Gibco) so that the final concentration was 200 µg/mL, 90 µL of the mixture was added to each well and allowed to stand for 10 min, amyloid β protein (Beta-Amyloid 1-42, AnaSpec) was mixed with Opti-MEM so that the concentration was 150 µM, 10 µL of the mixture was added to each well, and the cells were cultured at 37°C and 5%CO₂ for 72h to induce cell death. Except for performing the procedure described above, the MTT formazan production was measured as described above to calculate %relative cell survival (designated as "Aβ + Extract" in FIG. 1)

The results are shown in FIG. 1.

Comparing "Control" with "Aβ" in the drawing. 1, %relative cell survival was remarkably low in the latter compared to the former, indicating that cell death was induced by treatment with amyloid β protein.

Referring to "Aβ + M3,5G" in the drawing, %relative cell survival was significantly high compared to "Aβ," indicating that cell death due to amyloid β protein was inhibited by malvidin-3,5-diglucoside. Inhibition of cell death was also confirmed for malvidin (see "Aβ + M"), but not for malvidin-3-glucoside (see "Aβ + M3G").

Referring to "Aβ+ Extract" in the drawing, %relative cell survival was significantly high compared to "Aβ" and was comparable to that of "Control," indicating that a high inhibitory effect can be obtained when malvidin-3,5-diglucoside was used as an extract. High %relative cell survival observed for "Aβ+ Extract" suggests that malvidin-3,5-diglucoside may have cell proliferation effects in addition to cell death inhibitory effects.

### <Example 2: Gene Expression Experiment>

The mechanism of inhibitory effects of malvidin-3,5-diglucoside on neuronal cell death were analyzed by the experiment described below.

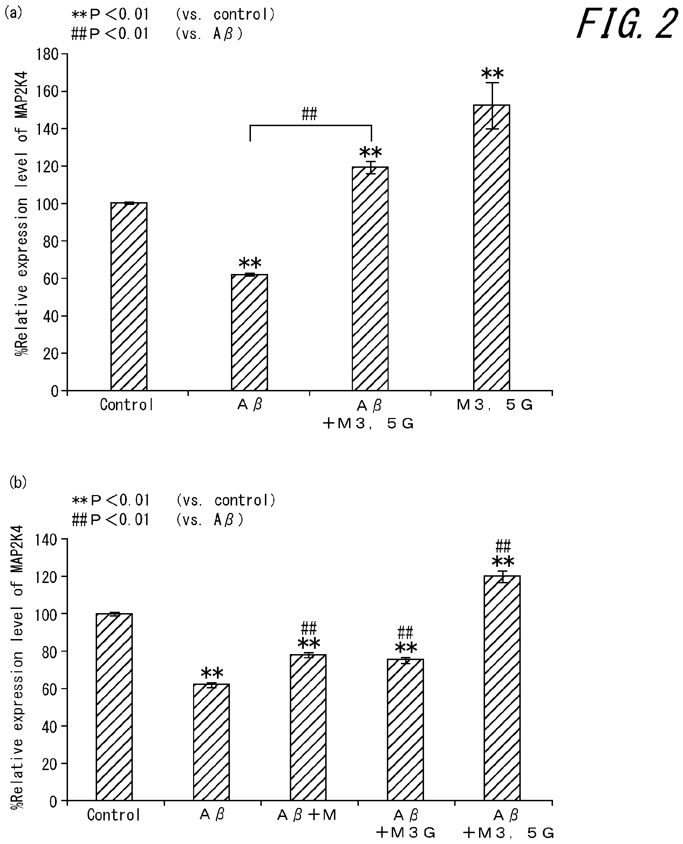

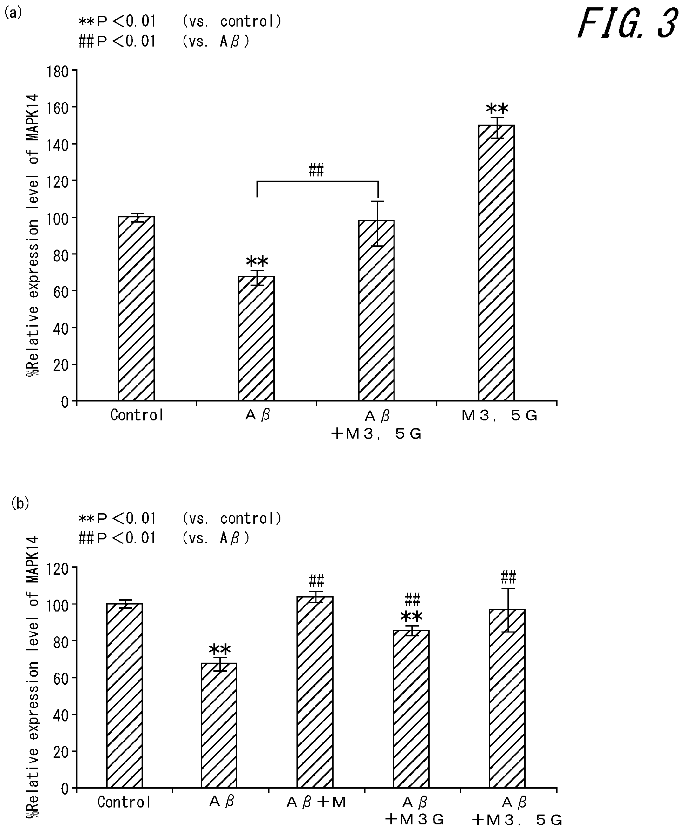

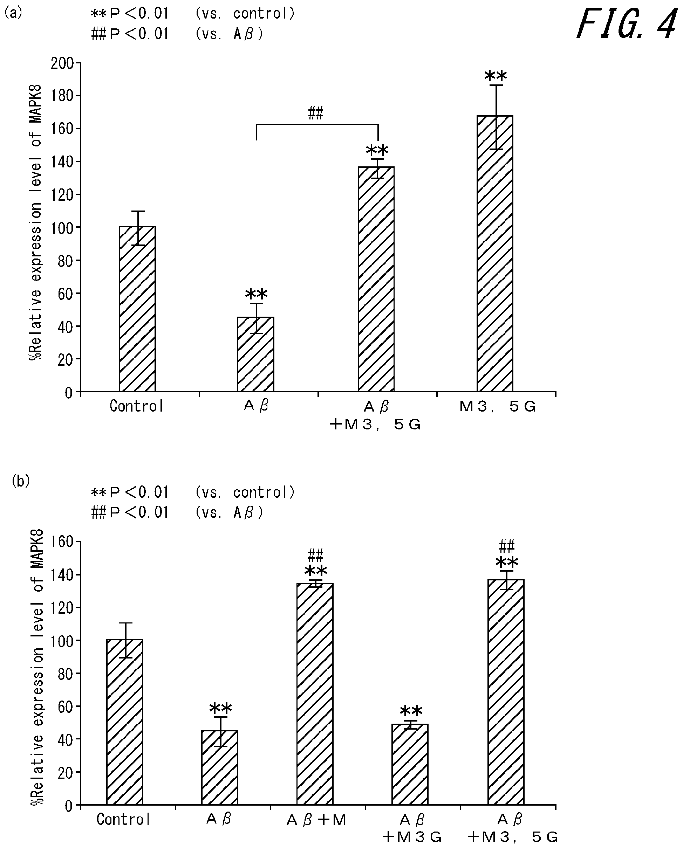

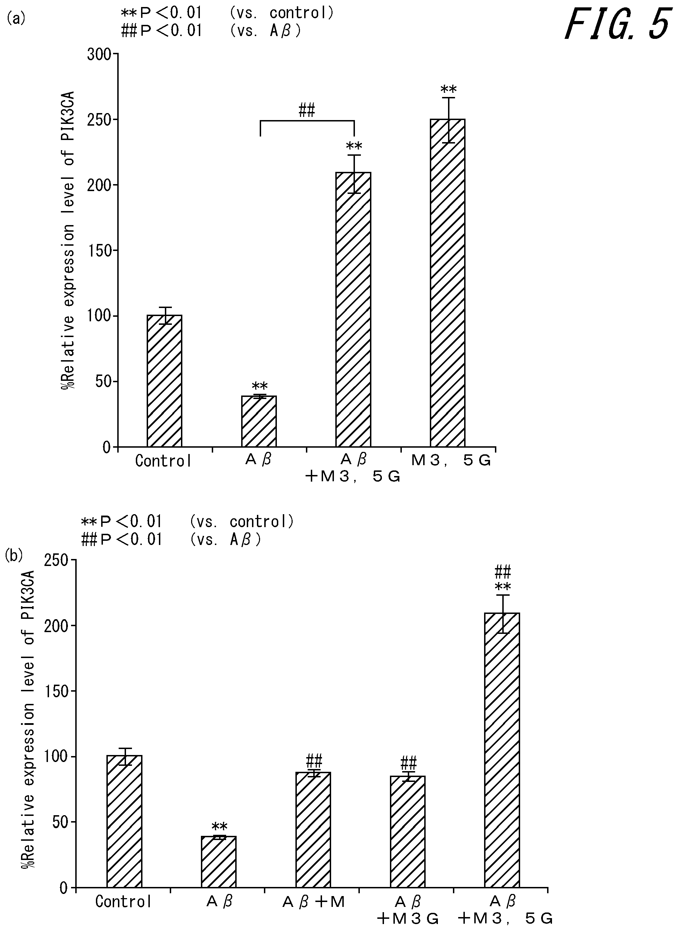

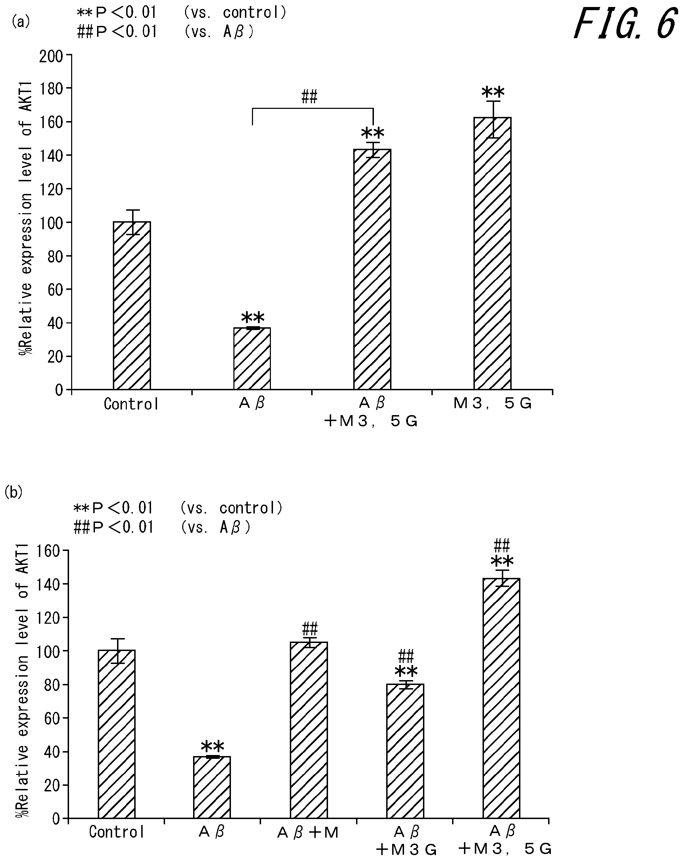

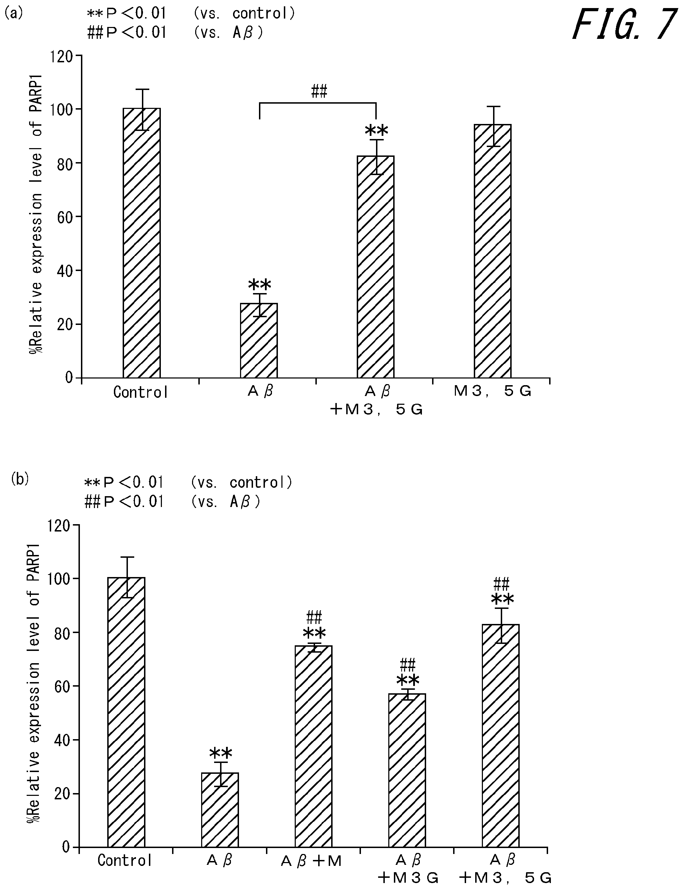

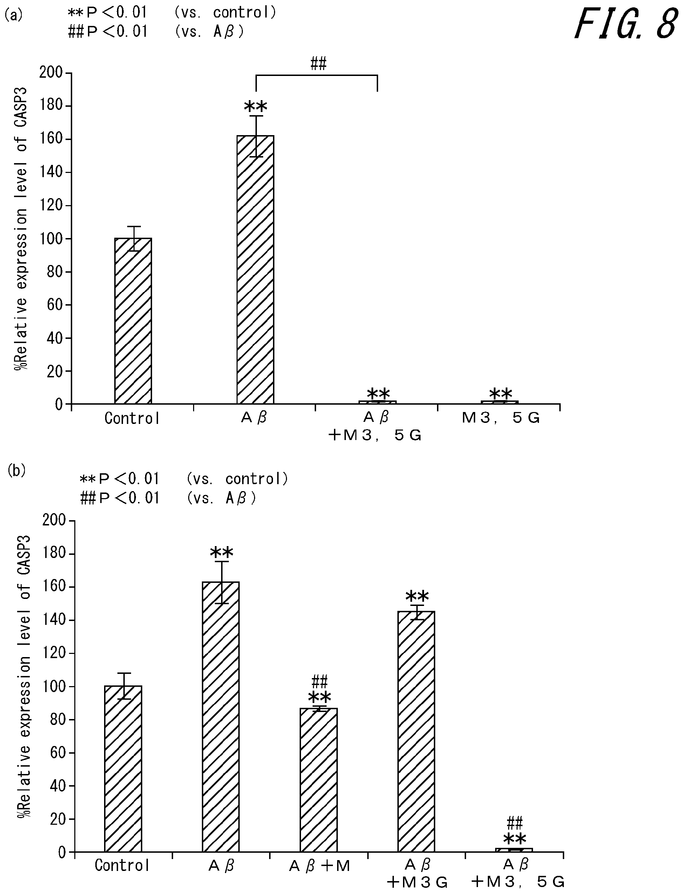

SH-SY5Y cells were seeded in 100 mm-cell dishes (BD Biocoat) at concentrations of 3.7×10⁵ cells/mL (10 mL/dish) and cultured for 24h at 37°C and 5%CO₂. Culture medium used was F12/DMEM (Gibco) supplemented with 1% non-essential amino acids (Gibco), 15%FBS (Bio West), and 1% penicillin/streptomycin (Lonza). After 24h, the culture medium was completely removed with an aspirator, 10 mL of culture medium Opti-MEM(Gibco) was added to each dish, and the cells were cultured for 24h at 37°C and 5%CO₂. RNA was extracted from the culture cells and analyzed for the expression of genes described below by real-time PCR (designated as "Control" in FIGS. 2 to 8).

RNA extraction was performed using ISOGEN Reagent (Nippon Gene Co., Ltd.) in accordance with the protocol recommended by Nippo n Gene Co., Ltd. (see URL: https://www.nippongene.com/siyaku/product/e xtraction/tds/tds_isogen-rna-dna-protein.pdf). Real-time PCR was performe d according to the protocol recommended by Thermo Fisher Scientific u sing a reagent kit for TaqMan Real-Time PCR (Thermo Fisher Scientifi c) (see URL: https://assets.thermofisher.com/TFS-Assets/LSG/manuals/430 4449_TaqManPCRMM_UG.pdf).
MAP2K4 (mitogen-activated protein kinase 4)
MAPK14 (mitogen-activated protein kinase 14)
MAPK8 (mitogen-activated protein kinase 8)
PI3KCA (phosphatidylinositol-4,5-bisphosphate 3-kinase catalyst subunit α)
AKT1 (AKT serine/threonine kinase 1)
PARP1 (poly(ADP-ribose) polymerase 1)
CASP3(Caspase-3)

These genes are involved in the stress-response MAPK pathway. These genes are known to be activated by various environmental stress stimuli such as ultraviolet rays, radiation, oxidative stress, or heat shock to induce cell death (apoptosis) in stressed cells. This signaling pathway is known to be also activated by amyloid β and play a pivotal role in the control of neurodegenerative diseases. There are a signaling pathway from a series of mitogen-activated protein kinase to PI3KCA and AKT1, and a signaling pathway from a series of mitogen-activated protein kinases to CASP3 and PARP1, with the former involved in mitochondrial activity and the latter involved in apoptosis.

24h after seeding 100 mm-cell dishes (BD Biocoat) with SH-SY5Y cells, the culture medium was completely removed with an aspirator, amyloid β protein (Beta-Amyloid 1-42, AnaSpec) was mixed with Opti-MEM (Gibco) so that the final concentration was 5 µM, 10 mL of the mixture was added to each dish, the cells were cultured for 24h at 37°C and 5%CO₂, and RNA was extracted. Except for performing the procedure described above, gene expression was analyzed as described above (designated as "Aβ" in FIGS. 2 to 8).

24h after seeding 100 mm-cell dishes (BD Biocoat) with SH-SY5Y cells, the culture medium was completely removed with an aspirator, Sample 1 was mixed with culture medium Opti-MEM(Gibco) so that the final concentration was 12.56 µM, 9,990 µL of the mixture was added to each dish and allowed to stand for 10 min, amyloid β protein (Beta-Amyloid 1-42, AnaSpec) was mixed with Opti-MEM (Gibco) so that the final concentration was 150 µM, 10 µL of the mixture was added to each well, the cells were cultured for 24h at 37°C and 5%CO₂, and RNA was extracted. Except for performing the procedure described above, Gene expression was analyzed as described above (designated as "Aβ + M3,5G" in FIGS. 2 to 8).

Except that Sample 2 was used instead of Sample 1, gene expression was analyzed as described above (designated as "Aβ + M" in FIGS. 2 to 8).

Except that Sample 3 was used instead of Sample 1, gene expression was analyzed as described above (designated as "Aβ + M3G" in FIGS. 2 to 8).

24h after seeding 100 mm-cell dishes (BD Biocoat) with SH-SY5Y cells, the culture medium was completely removed with an aspirator, 10 mL of culture medium Opti-MEM(Gibco) mixed with Sample 1 was added to each dish, the cells were cultured for 24h at 37°C and 5%CO₂, and RNA was extracted. Except for performing the procedure described above, gene expression was analyzed as described above (designated as "M3,5G" in FIGS. 2 to 8).

The results are shown in FIGS. 2 to 8.

Referring to "Aβ" in the drawing, the expression of MAP2K4, MAPK14, MAPK8, PI3KCA, AKT1 and PARP1 was significantly decreased and the expression of CASP3 was significantly increased compared to "Control."

On the other hand, referring to "M3,5G" in the drawing, the expression of MAP2K4, MAPK14, MAPK8, PI3KCA and AKT1 was significantly increased and the expression of CASP3 was significantly decreased compared to "Control" with the expression of PARP1 being comparable to that of "Control."

Referring to "Aβ + M3,5G" in the drawing, the expression of MAP2K4, MAPK14, MAPK8, PI3KCA, AKT1 and PARP1 was significantly high and the expression of CASP3 was significantly low compared to "Aβ." Further, the expression of MAP2K4, MAPK8, PI3KCA, and AKT1 of Aβ + M3,5G" was significantly high and the expression of CASP3 was significantly low compared to "Control."

These results suggest that malvidin-3,5-diglucoside has an effect of increasing the expression of MAP2K4, MAPK14, MAPK8, PI3KCA and AKT1 to restore decreased expression of these genes caused by amyloid β protein treatment and thereby contributes to the promotion of energy-metabolism by increasing mitochondrial activity.

These results also suggest that malvidin-3,5-diglucoside has an effect of decreasing the expression of CASP3 to prevent increased expression of CASP3 caused by amyloid β protein treatment and thereby suppresses apoptosis.

In the case of amyloid β protein-treated cells, on the other hand, there was an increase in the expression of MAP2K4, MAPK14, MAPK8, PI3KCA, AKT1, PARP1 and a decrease in the expression of CASP3 even when treated with malvidin (see "Aβ + M") and there was an increase in the expression of MAP2K4, MAPK14, PI3KCA,AKT1 and PARP1 even when treated with malvidin-3-glucoside (see "Aβ + M3G"), with malvidin-3,5-diglucoside showing the highest activity in increasing the expression of MAP2K4, PI3KCA and AKT1 and decreasing the expression of CASP3. These results indicate that activity increases in the order of malvidin-3-glucoside, and malvidin-3,5-diglucoside.

### <Example 3: ATP Production Measurement Experiment>

The ATP production in neuronal cells was measured by the experiment described below to confirm the promotion of energy metabolism by malvidin-3,5-diglucoside.

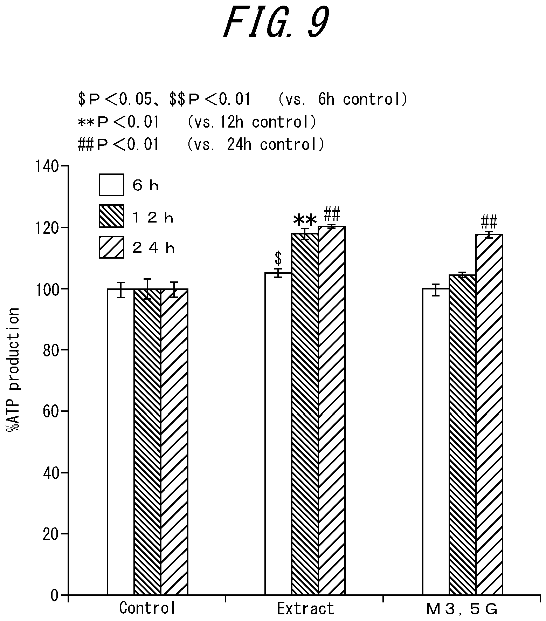

Specifically, ATP production was measured according to the protocol recommended by Toyo B-NET (URL: https://search.cosmobio.co.jp/cosmo_search_p/search_gate2/docs/TIC_/CA10 .20131125.pdf) using an ATP assay reagent ("Cell" ATP Assay reagent, Toyo B-NET) (designated as "Control" in FIG. 9).

Each well of a 96-well plate (BD Biocoat) was seeded with SH-SY5Y cells (model of neuronal cells, ATCC) at a concentration of 2×10⁵ cells/mL (100 µL/well) and the cells were cultured at 37°C and 5%CO₂ for 24h. Culture medium used was F12/DMEM (Gibco) supplemented with 1% non-essential amino acids (Gibco), 15%FBS (Bio West), and 1% penicillin/streptomycin (Lonza).

After 24h, the culture medium was completely removed with an aspirator, 100 µL of culture medium Opti-MEM (Gibco) was added to each well, and the cells were cultured for 6h, 12h, and 24h at 37°C and 5%CO₂. After each culturing time point, 100 µL of ATP assay reagent ("Cell" ATP Assay reagent, Toyo Ink)) was added to each well and mixed and the mixture was allowed to stand for reaction for 10 min at room temperature in dark conditions. 150 µL of the mixture solution of cells and ATP assay reagent in each well was transferred to a white 96-well plate (BD FALCON) using a micropipette. Light emission after a lapse of a predetermined time was measured on a microplate reader (Sumitomo Dainippon Pharma) to find ATP production (designated as "Control" in FIG. 9).

24h after seeding a 96-well plate (BD Biocoat) with SH-SY5Y cells, the culture medium was completely removed with an aspirator, Sample 1 was mixed with Opti-MEM (Gibco) so that the final concentration was 12.56 µM, 100 µL of the mixture was added to each well. Except for performing the procedure described above, ATP production was measured as described above (designated as "M3,5G" in FIG. 9).

24h after seeding a 96-well plate (BD Biocoat) with SH-SY5Y cells, the culture medium was completely removed with an aspirator, Sample 4 was mixed with Opti-MEM (Gibco) so that the concentration was 200 µg/mL, and 100 µL of the mixture was added to each well. Except for performing the procedure described above, ATP production was measured as described above (designated as "Extract" in FIG. 9).

The results are shown in FIG. 9.

Both "M3,5G" and "Extract" showed increased ATP production compared to "Control," suggesting that energy metabolism was promoted due to increased mitochondrial activity by malvidin-3,5-diglucoside.

### <Example 4: Reactive Oxygen Species Measurement Experiment>

The reactive oxygen species level in neuronal cells was measured by the experiment described below to confirm oxidative stress reduction by malvidin-3,5-diglucoside.

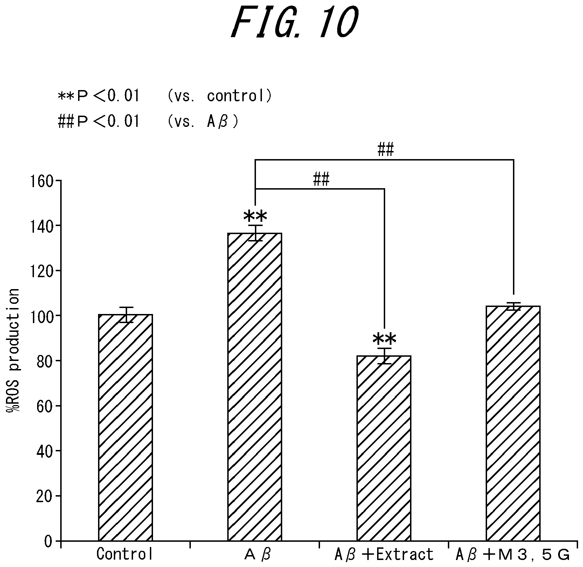

Specifically, OxiSelect^{™} Intracellular ROS assay kit (Cell Biolabs Ink) was used to measure the reactive oxygen species level according to protocol recommended by Cell Biolabs Ink (URL: https://www.cellbiolabs.com/sites/default/files/STA-342-ROS-assay-kit.pdf)) of Cell Biolabs Ink Co., Ltd. (designated as "Control" in FIG. 10).

Each well of a 96-well plate (BD Biocoat) was seeded with SH-SY5Y cells (ATCC, model of neuronal cells) at a concentration of 2×10⁵ cells/mL (100 µL/well) and the cells cultured at 37°C and 5%CO₂ for 24h. Culture medium used was F12/DMEM (Gibco) supplemented with 1% non-essential amino acids (Gibco), 15%FBS (Bio West), and 1% penicillin/streptomycin (Lonza).

After 24h, the culture medium was completely removed with an aspirator, 20× DCFH-DA (reagent included in the assay kit) was mixed with culture medium Opti-MEM (Gibco) (reagent was diluted 20 times), 100 µL of the mixture was added to each well, and the cells were cultured at 37°C and 5%CO₂ for 1h. The culture medium Opti-MEM with 20× DCFH-DA was then completely removed with an aspirator, 100 µ of fresh Opti-MEM (Gibco) was added to each well, and the cells were cultured at 37°C and 5%CO₂ for 1h. 100 µL of 2× Cell Lysis Buffer (reagent included in the assay kit) was added to each well and mixed and the mixture was allowed to stand for reaction for 10 min at room temperature under dark conditions. 150 µL of the reaction solution in each well was transferred to another 96-well plate (BD FALCON) using a micropipette. Fluorescence at 480 nm/530 nm was measured on a microplate reader (Sumitomo Dainippon Pharma) to find the relative ROS production (designated as "Control" in FIG. 10).

1h after treatment with Opti-MEM (Gibco) containing 20× DCFH-DA (reagent included in the assay kit), the culture medium was completely removed with an aspirator, amyloid β protein (Beta-Amyloid 1-42, AnaSpec) was mixed with Opti-MEM (Gibco) so that the final concentration was 5 µM, 100 µL of the mixture was added to each well and the cells were cultured at 37°C and 5%CO₂ for 1h. Except for performing the procedure described above, the reactive oxygen species level was obtained as described above (designated as "Aβ" in FIG. 10).

1h after treatment with Opti-MEM (Gibco) containing 20× DCFH-DA (reagent included in the assay kit), the culture medium was completely removed with an aspirator, Sample 1 and amyloid β protein (Beta-Amyloid 1-42, AnaSpec) were mixed with Opti-MEM (Gibco) so that the final concentration was 12.56 µM and 5 µM, respectively, 100 µL of the mixture was added to each well and the cells were cultured at 37°C and 5%CO₂ for 1h. Except for performing the procedure described above, the reactive oxygen species level was obtained as described above (designated as "Aβ + M3,5G" in FIG. 10).

1h after treatment with Opti-MEM (Gibco) containing 20× DCFH-DA (reagent included in the assay kit), the culture medium was completely removed with an aspirator, Sample 4 and amyloid β protein (Beta-Amyloid 1-42, AnaSpec) were mixed with Opti-MEM (Gibco) so that the final concentration was 12.56 µM and 5 µM, respectively, 100 µL of the mixture was added to each well and the cells were cultured at 37°C and 5%CO₂ for 1h. Except for performing the procedure described above, the reactive oxygen species level was obtained as described above (designated as "Aβ + Extract" in FIG. 10).

The results are shown in FIG. 10.

"Aβ" showed significant increases in ROS production compared to "Control." This suggests an increase in oxidative stress in "Aβ." On the other hand, "Aβ + M3,5G" and "Aβ + Extract" showed significant reductions in ROS production compared to "Aβ" but no significance was observed when compared to "Control," suggesting that treatment with the compound M3,5G or the extract alleviates Aβ-induced oxidative stress to a level of Control (condition without oxidative stress).

### <Example 5: Animal Experiment>

Effects of malvidin-3,5-diglucoside on improving learning and memory ability in animals were confirmed by the experiment described below.

Mice and administration regimens used in the experiment are as described below. Breeding conditions are common to all groups.

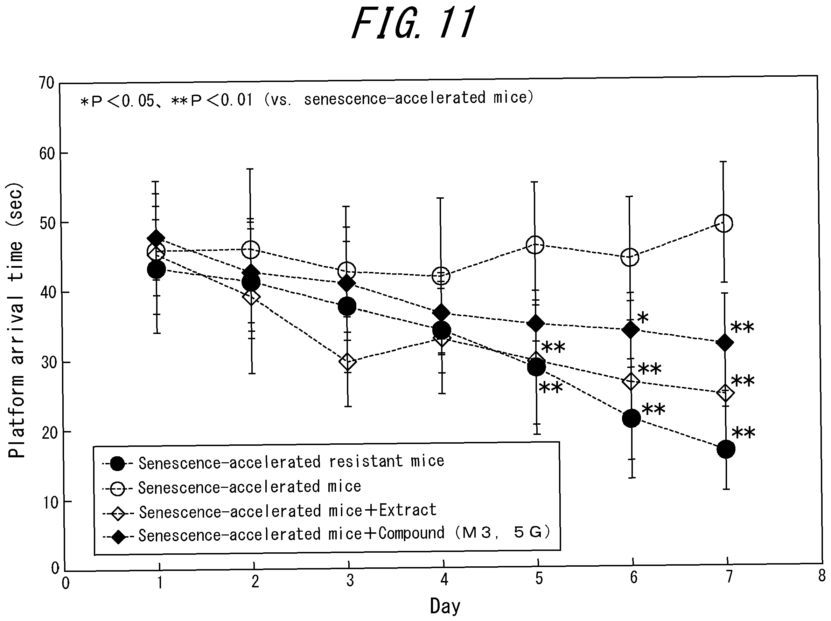

Compound (M3,5G)-treated (SAMP8) group: Sample 5 was orally administered to 10 senescence-accelerated mice (SAMP8) (16 weeks old, weighing 28-30 g, Japan SLC, Inc.) for a period of 30 days at a daily dose of 2.17 mg of malvidin-3,5-diglucoside per kg of mouse body weight (designated as "Senescence-accelerated mice + Compound (M3,5G)" in FIGS. 11 to 13).

The human equivalent dose for 2.17 mg of malvidin-3,5-diglucoside per kg of mouse body weight corresponds to 0.18 mg of malvidin-3,5-diglucoside per kg of human body weight.

Extract-treated (SAMP8) group: Sample 4 was orally administered to 10 senescence-accelerated mice (SAMP8) (16 weeks old, weighing 28-30 g, Japan SLC, Inc.) for a period of 30 days at a daily dose of 25 mg of extract powder (2.17 mg of malvidin-3,5-diglucoside in Sample 6) per kg of mouse body weight (designated as "Senescence-accelerated mice + Extract" in FIGS. 11 to 13).

Water-treated (SAMP8) group: drinking water of almost the same volume as that of Sample 5 or 6 administered to compound (M3,5G)-treated group or extract-treated group was orally administered to 10 senescence-accelerated mice (SAMP8) (16 weeks old, weighing 28-30 g, Japan SLC, Inc.) for a period of 30 days (designated as "Senescence-accelerated mice" in FIGS. 11 to 13).

Water-treated (SAMR1) group: drinking water of almost the same volume as that of Sample 5 or 6 administered to compound (M3,5G)-treated group or extract-treated group was orally administered to 10 senescence-accelerated resistant mice (SAMR1) (16 weeks old, weighing 28.5-34 g, Japan SLC, Inc.) for a period of 30 days (designated as "Senescence-accelerated resistant mice" in FIGS. 11 to 13).

The content of the animal experiment is as described below.

From the day following the completion of oral administration, mice in each group were subjected to the Morris water maze test (platform test, probe test). A water pool having a diameter of 120 cm and a depth of 45 cm was prepared as the Morris water maze. A transparent platform was placed under the water surface of the pool. On the rim of the pool were four marks placed at equal intervals.

### Platform Test:

Mice were released into the pool and the time taken for the mice to arrive at the platform was measured. The time limit was set to 60 sec, with the arrival time when the mouse failed to arrive at the platform within 60 sec set to 60 sec. After arrival the mice were allowed to remain on the platform for 15 sec. The mouse from each group was subjected to this experiment once a day for 7 consecutive days. The results are shown in FIG. 11.

### Probe Test:

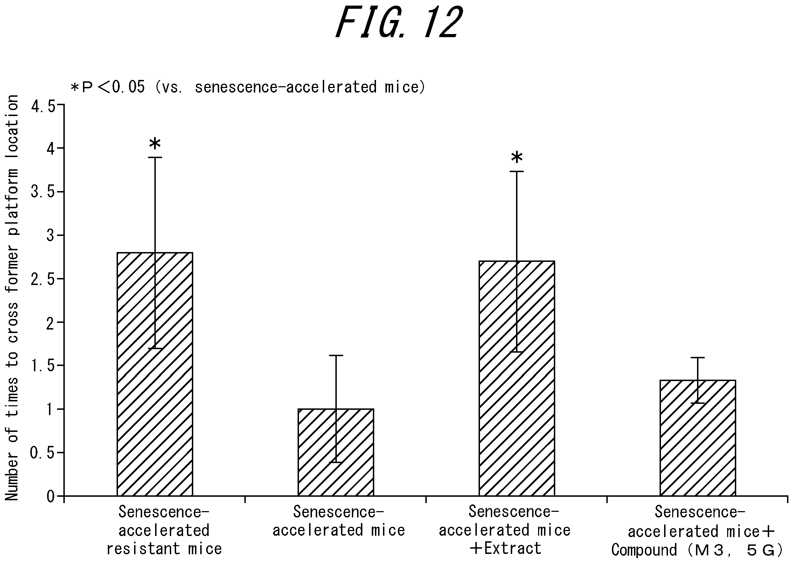

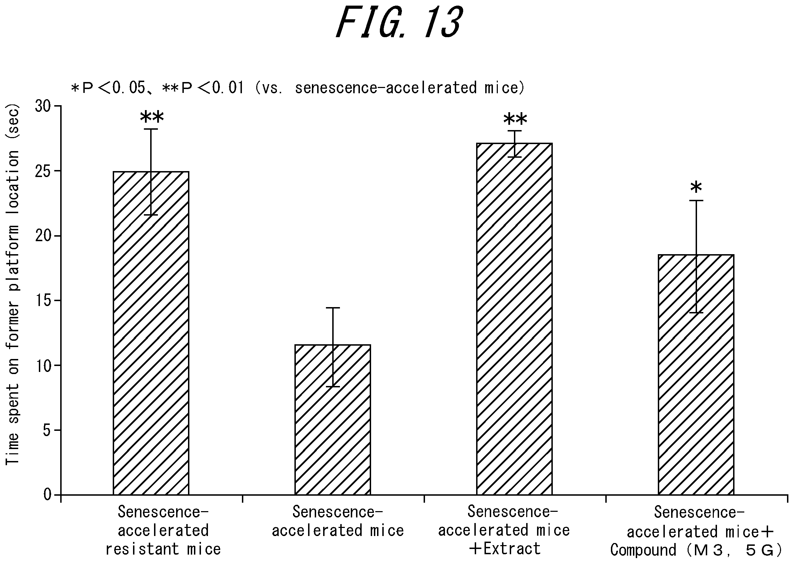

On the last day of the test, the platform was removed from the pool and the mice were again released into the pool. The number of times the mouse crossed the former platform location was counted and the time the mouse spent on the former platform location was measured. The results are shown in FIGS. 12 and 13.

The platform test assesses the spatial learning and memory of a mouse based on the time it took for the mouse to arrive at the platform by relying on visual stimuli such as landmarks. As shown in FIG. 11, the arrival time was shortened for senescence-accelerated resistant mice (water-treated), whereas the arrival time was not shortened for senescence-accelerated mice (water-treated). Senescence-accelerated mice treated with the extract exhibited arrival time shortening comparable to that for senescence-accelerated resistant mice (water-treated). When compared with senescence-accelerated mice treated with malvidin-3,5-diglucoside, senescence-accelerated mice treated with the extract exhibited greater arrival time shortening.

The probe test assesses the memory retention ability of a mouse by the number of times the mouse crossed the former platform location and the time spent on the former platform location. As shown in FIGS. 12 and 13, senescence-accelerated mice (water-treated) crossed the former platform location significantly fewer times and spent shorter time on the former platform location compared to senescence-accelerated resistant mice (water-treated). For senescence-accelerated mice treated with the extract, the number of the times the mouse crossed the former platform location and the time spent on the former platform location were comparable to those for senescence-accelerated resistant mice (water-treated). When compared with senescence-accelerated mice treated with malvidin-3,5-diglucoside, senescence-accelerated mice treated with the extract crossed the former platform location more times and spent longer time on the former platform location

The above results confirm improvements in learn and memory ability of animals by the administration of malvidin-3,5-diglucoside, and suggest that high improvement effects are obtained especially in the case of extract administration.

### INDUSTRIAL APPLICABILITY

The neuronal function modulating agent of the present disclosure is effective for neuronal cell protection and exerts neuronal function modulating effects through neuronal cell protection, and can be expected to improve and prevent neurodegenerative diseases such as Alzheimer's disease, Parkinson's disease, and depression, and is therefore expected to be used for health supplements and other foods, pharmaceuticals etc. to expand the related markets. Because the neuronal function modulating agent of the present disclosure comprises as an active ingredient malvidin-3,5-diglucoside which is natural origin, it is considered safe and suitable for daily or continuous ingestion. Thus, the present disclosure has high industrial utility.

## Claims

1. A neuronal function modulating agent comprising malvidin-3,5-diglucoside as an active ingredient.

2. The neuronal function modulating agent according to claim 1, wherein the neuronal function modulating agent exerts a neuronal function modulating effect through neuronal cell protection.

3. The neuronal function modulating agent according to claim 2, wherein the neuronal cell protection comprises suppressing neuronal cell death caused by amyloid β protein.

4. A neuronal function modulating agent according to any one of claims 1 to 3, comprising delphinidin-3-glucoside.

5. The neuronal function modulating agent according to any one of claims 1 to 4, wherein the neuronal function modulating agent is for oral administration.

6. A pharmaceutical composition comprising the neuronal function modulating agent according to any one of claims 1 to 5.

7. A food composition comprising the neuronal function modulating agent according to any one of claims 1 to 5.

8. The food composition according to claim 7, wherein the food composition is a functional food, a dietary supplement, a health supplement, a nutritionally fortified food, a food with a functional claim, a food for special dietary use, a food for specified health use, or a food with a nutritional function.

9. A method of a modulating neuronal function, comprising ingesting the neuronal function modulating agent according to any one of claims 1-5 into a subject.
